# FASCICULE DE BREVET EUROPEEN

(11) **EP 0 952 147 B1**
(45) Date de publication et mention de la délivrance du brevet: **29.08.2001**
(21) Numéro de dépôt: 99400724.3
(22) Date de dépôt: 25.03.1999
(51) Int. Cl.: C07C 309/80, C07C 303/10, B01J 19/12

(54) **Procédé de sulfochloration photochimique d'alcanes gazeux**
Verfahren zur photochemischen Sulfochlorierung von gasförmigen Alkanen
Process for the photochemical sulfochlorination of gaseaous alkanes

(30) Priorité: 21.04.1998 FR 9804961
(43) Date de publication de la demande: 27.10.1999
(73) Titulaire: Atofina, 92800 Puteaux (FR)
(72) Inventeur: Ollivier, Jean, 64260 Arudy (FR)
(74) Mandataire: Leboulenger, Jean

(56) Documents cités:
- DE-A- 3 708 784
- DE-B- P20 023
- FR-A- 2 578 841
- FR-A- 2 595 095
- US-A- 4 997 535

## Description

La présente invention concerne le domaine des chlorures d'alcanesulfonyle et a plus particulièrement pour objet la fabrication de ces composés par sulfochloration photochimique des alcanes gazeux à la température ambiante.

Etant donné l'utilité industrielle des chlorures d'alcanesulfonyle, en particulier du chlorure de méthanesulfonyle, la fabrication de ces composés a fait l'objet de plusieurs procédés consistant notamment en la sulfochloration photochimique d'alcanes avec le chlore et le dioxyde de soufre. Parmi ces procédés connus, un procédé particulièrement performant pour la sulfochloration photochimique des alcanes gazeux à la température ambiante comme le méthane est celui décrit dans les brevets FR 2 578 841 et FR 2 595 095.

Ce procédé qui consiste essentiellement à faire réagir un mélange gazeux d'alcane, de dioxyde de soufre et de chlore en présence de lumière ultraviolette fournie par une lampe au mercure, est caractérisé en ce que le mélange contient un fort excès de dioxyde de soufre par rapport à l'alcane et que du dioxyde de soufre liquide est injecté dans la zone de réaction pour maintenir constante la température de celle-ci. Une installation pour la mise en oeuvre de ce procédé est également décrite dans les brevets précités dont le contenu est incorporé ici par référence.

Par rapport aux procédés photochimiques de la technique antérieure, décrits dans l'ouvrage de F.ASINGER "Paraffines, Chemistry and Technology", Pergamon Press 1968, p.520 et suivantes et dans le brevet FR 2 246 520, le procédé des brevets FR 2 578 841 et FR 2 595 095 présente l'avantage de n'exiger l'introduction d'aucun produit étranger dans le milieu réactionnel et de former ce dernier uniquement avec ses constituants obligatoires, à savoir l'alcane, le dioxyde de soufre et le chlore. D'autre part, ce procédé permet d'obtenir de bonnes conversions et des rendements satisfaisants aussi bien par rapport à l'alcane que par rapport au chlore. En outre, contribuant à une meilleure absorption des photons par le chlore et à une élimination très aisée de la chaleur de réaction, ce procédé conduit à d'excellents rendements quantiques et évite toute surchauffe du milieu réactionnel.

Il a maintenant été trouvé qu'on peut encore améliorer ces performances en utilisant comme source lumineuse une lampe au mercure dopée au gallium. Par rapport à une lampe au mercure d'égale puissance, l'emploi d'une telle source lumineuse permet d'obtenir une productivité du réacteur nettement supérieure, ainsi qu'une amélioration du rendement et de la sélectivité de la réaction.

L'invention a donc pour objet un procédé de fabrication de chlorures d'alcanesulfonyle par réaction photochimique d'un alcane avec du chlore et du dioxyde de soufre, éventuellement en présence de chlorure d'hydrogène, caractérisé en ce que l'on utilise comme source lumineuse une lampe au mercure moyenne pression dopée au gallium.

Le procédé selon l'invention vise plus particulièrement la sulfochloration du méthane qui est l'alcane le plus difficile à sulfochlorer, mais il s'applique également à tous les alcanes gazeux dans les conditions de température et de pression choisies.

Selon l'alcane de départ, les proportions des réactifs dans le mélange gazeux soumis au rayonnement lumineux peuvent varier entre les limites suivantes :

| | par mole de méthane | par mole d'alcane en C₂ ou plus |
|---|---|---|
| SO₂ | 1 à 12 moles | 7 à 14 moles |
| Cl₂ | 0,1 à 1 mole | 0,1 à 1 mole |
| HCI | 0,1 à 0,6 mole | 0 |

et sont de préférence choisies comme suit :

| | | |
|---|---|---|
| SO₂ | 5 à 7 moles | 10 à 13 moles |
| Cl₂ | 0,7 à 0,9 mole | 0,7 à 0,9 mole |
| HCI | 0,4 à 0,5 mole | 0 |

On opère de préférence sous une pression supérieure à la pression atmosphérique. Généralement, cette pression peut aller de 1 à 15 bars relatif et est, de préférence, comprise entre 8 et 12 bars relatifs.

La température réactionnelle, généralement comprise entre 10 et 90°C, dépend de la pression de travail choisie. Elle est par exemple d'environ 60°C pour 10 bars absolus et d'environ 80°C pour 15 bars absolus. Comme dans le procédé décrit dans les brevets FR 2 578 841 et FR 2 595 095, la température est maintenue constante par injection de SO₂ liquide dans la zone réactionnelle.

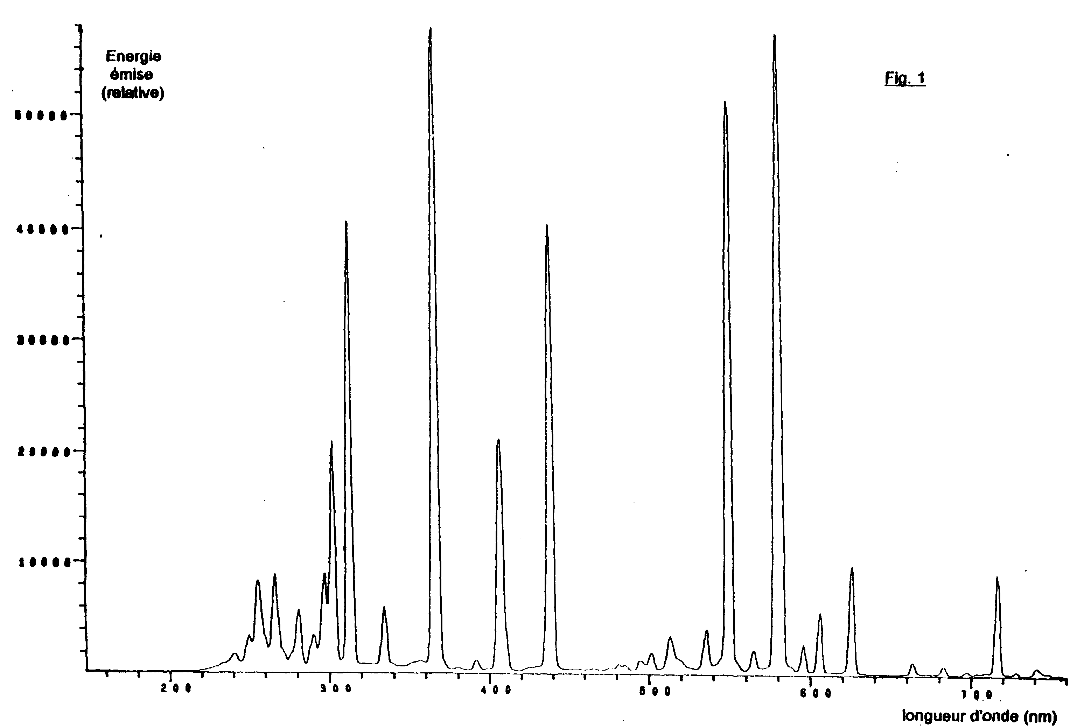

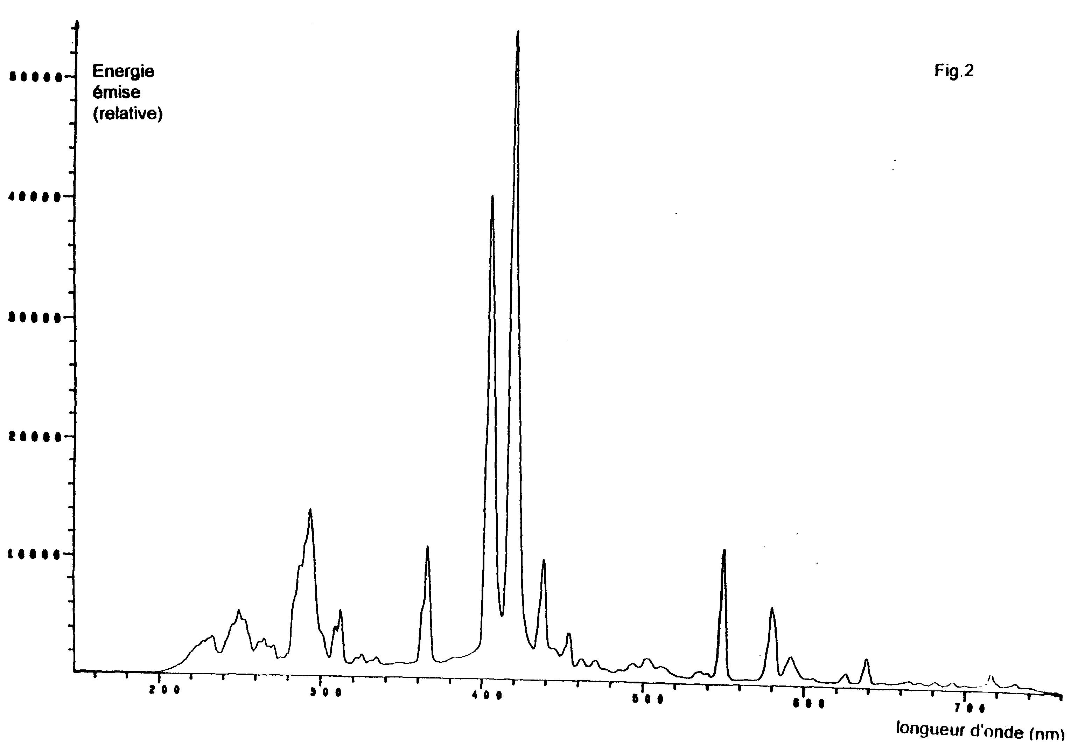

Les lampes au mercure moyenne pression dopées au gallium à utiliser conformément au procédé selon l'invention sont bien connues et sont décrites, par exemple, au chapitre 9 de l'ouvrage de R.Philips intitulé "Sources and Applications of Ultraviolet Radiation" Academic Press 1983, p.264-276 dont le contenu est incorporé ici par référence. De telles lampes, commercialisées par les Sociétés HERAEUS, SILITRO/SCAM et PHILIPS, réemettent plus de 60 % de leur énergie lumineuse sous forme de radiations de longueurs d'onde comprises entre 400 et 485 nm. Les figures 1 et 2 annexées montrent respectivement le spectre d'émission d'une lampe au mercure moyenne pression de 750 watts et celui d'une lampe au mercure moyenne pression de même puissance dopée au gallium. L'énergie lumineuse émise par la lampe au mercure moyenne pression (figure 1) est répartie sous forme de raies entre 220 et 750 nm alors que, pour la lampe dopée au gallium (figure 2), l'essentiel de l'énergie émise est concentrée dans la zone de 400 à 430 nm. Outre un gain de rendement d'énergie lumineuse utile (environ 40 %), l'éclairement du milieu réactionnel avec une lampe au mercure moyenne pression dopée au gallium est beaucoup plus homogène qu'avec une lampe au mercure classique. Ceci contribue à un amorçage de la réaction mieux réparti dans le volume réactionnel et, en favorisant les transferts thermiques, permet d'atténuer les surchauffes locales liées à l'énergie de la réaction ; on observe donc une meilleure sélectivité.

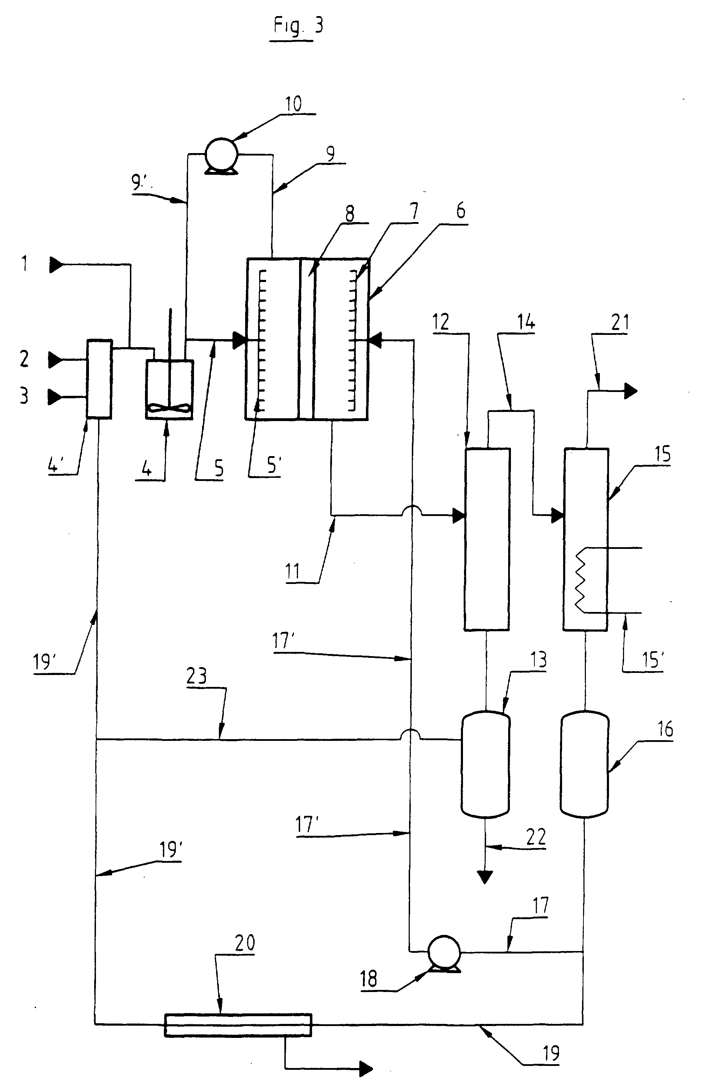

Le procédé selon l'invention peut être mis en oeuvre dans une installation similaire à celle décrite dans le brevet FR 2 578 841. Une telle installation comprenant essentiellement des moyens d'alimentation des réactifs, un réacteur photochimique et des moyens pour séparer les produits de la réaction est représentée par le dessin schématique de la figure 3 annexée.

Sur ce dessin, les entrées 1, 2 et 3 sont respectivement celles de l'alcane, du dioxyde de soufre et du chlore que l'on introduit à l'état gazeux dans un mélangeur 4 muni d'un agitateur pour homogénéiser le mélange gazeux ; pour des raisons de sécurité, un prémélangeur de Cl₂ et SO₂ est de préférence prévu en 4'. Le mélange gazeux passe du mélangeur 4 via la conduite 5 dans le réacteur 6 dans lequel il est distribué uniformément au moyen d'une rampe 5' à orifices. Une autre rampe similaire 7 est placée également suivant la hauteur du réacteur pour introduire le SO₂ liquide destiné au réglage de la température. Le réacteur est traversé de manière connue en soi par une source lumineuse 8. En haut du réacteur 6 part une canalisation 9 vers une pompe 10, permettant de recycler une fraction de l'effluent du réacteur vers la canalisation 5 en vue de la prédilution des réactifs venant de 4. Une tubulure 11 conduit le produit liquide, formé dans le réacteur 6, vers un séparateur 12 d'où la phase liquide, c'est-à-dire le chlorure d'alcanesulfonyle brut, descend dans un stockage intermédiaire 13, tandis que les gaz résiduels passent par une conduite 14 dans un second séparateur 15. Ce séparateur est éventuellement muni d'un refroidisseur 15' pour ramener le SO₂, arrivant, à l'état liquide ; le SO₂ liquide contenant du chlore, est récupéré dans un stockage intermédiaire 16. Une fraction de SO₂ est recyclée par les canalisations 17 et 17' via la pompe 18 et la rampe 7 dans le réacteur 6. Une autre fraction de SO₂, venant de 16, passe par la canalisation 19 dans le réchauffeur 20 et de là par 19' vers l'alimentation du mélangeur 4.

En haut du séparateur 15, le HCI est évacué par la conduite 21 vers des appareils de traitement non représentés. Du bas du stockage intermédiaire 13 part une conduite 22 vers des appareils de purification du chlorure d'alcanesulfonyle produit qui, ne faisant pas l'objet de l'invention, ne sont pas représentés ici.

Les exemples suivants illustrent l'invention sans la limiter.

### EXEMPLE 1 (comparatif)

Dans le dispositif décrit précédemment, on a préparé du chlorure de méthanesulfonyle (CH₃SO₂Cl) en utilisant comme source lumineuse une lampe au mercure moyenne pression. Cette lampe de 750 watts était placée axialement dans un réacteur 6 de 50 litres de capacité.

Le mélange gazeux, préparé en 4, contenait pour une mole de méthane, 6,25 moles de dioxyde de soufre, 0,83 mole de chlore et 0,417 mole de chlorure d'hydrogène. Ce mélange gazeux était alimenté au réacteur au débit de 5,75 Nm³/heure. La pression dans le réacteur étant fixée à 9 bars au-dessus de l'atmosphère, la température était réglée à 65 ± 2°C par injection, au moyen de la rampe 7, de 5,1 kg/h de SO₂ liquide.

La quantité horaire de chlorure de méthanesulfonyle brut, recueilli après détente dans le stockage 13, était de 2,5 kg. A la pression atmosphérique et à la température ambiante, ce produit brut présentait la composition pondérale suivante :

| **Constitutant** | **% poids** |
|---|---|
| CH₃SO₂Cl | 76,5 |
| SO₂ | 18,4 |
| CH₃Cl | 0,5 |
| CH₂Cl₂ | 1,5 |
| CHCl₃ | 2,0 |
| CCl₄ | 0,1 |
| lourds | 1 |

L'effluent gazeux, arrivant par 14 dans le second séparateur 15 présentait la composition volumique suivante :

| **Constitutant** | **% volume** |
|---|---|
| SO₂ | 83,06 |
| CH₄ | 4,33 |
| HCl | 11,1 |
| Cl₂ | 1,0 |
| CH₃Cl | 0,5 |

Le débit de cet effluent gazeux était de 6,57 Nm³/h et contenait le SO₂ gazeux issus de l'évaporation ayant servi au refroidissement de la réaction. Afin de recueillir le dioxyde de soufre à l'état liquide sous 4 bars de pression relative, la température dans le séparateur 15 était maintenue au-dessous de 32°C.

Le débit de méthane à la sortie 21 du séparateur 15 était de 0,278 Nm³/heure. La quantité introduite en 1 étant de 0,68 Nm³/h, la conversion du méthane a donc été de 59 %. Pour le chlore, la conversion s'est élevée à 88 %.

Les résultats ont conduit aux rendements et sélectivité suivants en chlorure de méthanesulfonyle produit :

| | **Rendement (%)** | **Sélectivité (%)** |
|---|---|---|
| sur CH₄ | 55 | 93 |
| sur Cl₂ | 70 | 80,6 |

Ramenée à la puissance de la lampe au mercure moyenne pression, la productivité en chlorure de méthanesulfonyle a été de 2,55 kg/kW.

### EXEMPLE 2

Dans le même appareillage qu'à l'exemple 1, on a préparé du chlorure de méthanesulfonyle en remplaçant la lampe au mercure classique par une lampe dopée au gallium de même puissance électrique (750 W).

Afin d'avoir le même taux de conversion du chlore qu'à l'exemple 1 (88 %), le débit horaire du mélange gazeux d'alimentation a dû être amené à 6,86 Nm³/heure. La pression dans le réacteur étant fixée à 9 bars au-dessus de l'atmosphère, la température a été réglée à 65 ± 2°C par injection, au moyen de la rampe 7, de 7,5 kg/h de dioxyde de soufre liquide.

La quantité horaire de chlorure de méthanesulfonyle brut, recueilli après détente dans le stockage 13, était de 3,54 kg. A la pression atmosphérique et à la température ambiante, ce produit brut présentait la composition pondérale suivante :

| **Constitutant** | **% poids** |
|---|---|
| CH₃SO₂Cl | 76 |
| SO₂ | 21,15 |
| CH₃Cl | 0,4 |
| CH₂Cl₂ | 0,6 |
| CHCl₃ | 0,8 |
| CCl₄ | 0,05 |
| lourds | 1 |

L'effluent gazeux, arrivant par 14 dans le second séparateur 15 présentait la composition volumique suivante :

| **Constitutant** | **% volume** |
|---|---|
| SO₂ | 84,6 |
| CH₄ | 3,17 |
| HCl | 10,81 |
| Cl₂ | 0,92 |
| CH₃Cl | 0,5 |

Le débit de cet effluent gazeux, contenant le SO₂ gazeux issu de l'évaporation ayant servi au refroidissement de la réaction, était de 8,3 Nm³/h. Afin de recueillir le dioxyde de soufre à l'état liquide sous 4 bars de pression relative, la température dans le séparateur 15 était maintenue au-dessous de 32°C.

Le débit de méthane à la sortie 21 du séparateur 15 était de 0,26 Nm³/heure. La quantité introduite en 1 étant de 0,8 Nm³/h, la conversion du méthane a donc été de 67 %. Pour le chlore, la conversion s'est élevée à 88 %.

Les résultats ont conduit aux rendements et sélectivités suivants en chlorure de méthanesulfonyle produit :

| | **Rendement (%)** | **Sélectivité (%)** |
|---|---|---|
| sur CH₄ | 64,3 | 95,5 |
| sur Cl₂ | 76 | 86,4 |

Ramenée à la puissance de la lampe au gallium, la productivité en chlorure de méthanesulfonyle a été de 3,58 kg/kW.

Le tableau suivant résume les résultats des exemples précédents :

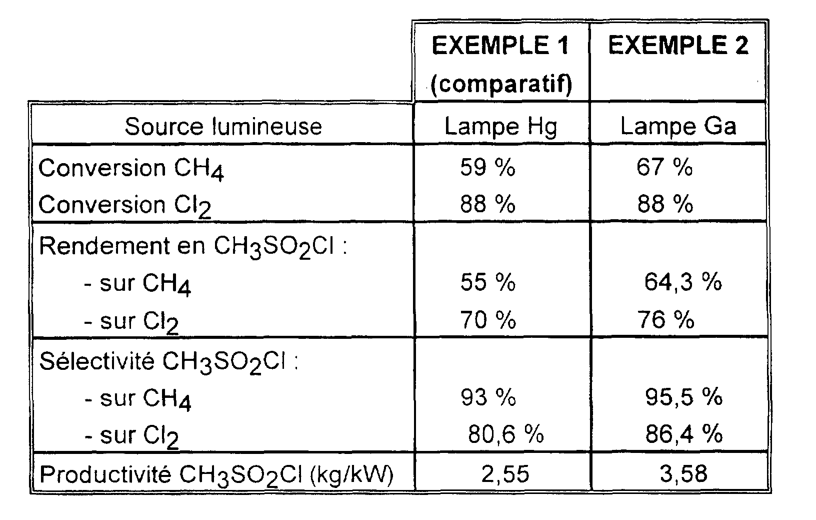

## Revendications

1. Procédé de fabrication de chlorures d'alcanesulfonyle par réaction photochimique d'un alcane avec du chlore et du dioxyde de soufre, éventuellement en présence de chlorure d'hydrogène, **caractérisé en ce que** l'on utilise comme source lumineuse une lampe au mercure moyenne pression dopée au gallium.

2. Procédé selon la revendication 1 dans lequel on opère sous une pression allant de 1 à 15 bars relatifs, de préférence comprise entre 8 et 12 bars relatifs.

3. Procédé selon la revendication 1 ou 2 dans lequel la température réactionnelle est comprise entre 10 et 90°C et maintenue constante par injection de SO₂ liquide dans la zone réactionnelle.

4. Procédé selon l'une des revendications 1 à 3 dans lequel l'alcane est le méthane, le mélange gazeux alimenté au réacteur comprenant 1 à 12 moles de dioxyde de soufre, 0,1 à 1 mole de chlore et 0,1 à 0,6 mole de chlorure d'hydrogène par mole de méthane.

5. Procédé selon la revendication 4 dans lequel le mélange gazeux contient 5 à 7 moles de dioxyde de soufre, 0,7 à 0,9 mole de chlore et 0,4 à 0,5 mole de chlorure d'hydrogène par mole de méthane.

6. Procédé selon l'une des revendications 1 à 3 dans lequel l'alcane contient au moins 2 atomes de carbone, le mélange gazeux alimenté au réacteur comprenant 7 à 14 moles (de préférence 10 à 13 moles) de dioxyde de soufre et 0,1 à 1 mole de chlore (de préférence 0,7 à 0,9 mole) par mole d'alcane.

## Patentansprüche

1. Verfahren zur Herstellung von Alkansulfonylchloriden durch photochemische Reaktion eines Alkans mit Chlor und Schwefeldioxid, gegebenenfalls in Gegenwart von Chlorwasserstoff, **dadurch gekennzeichnet**, daß man als Lichtquelle eine mit Gallium angereicherte Quecksilber-Mitteldrucklampe verwendet.

2. Verfahren nach Anspruch 1, in dem man bei einem Relativdruck von 1 bis 15 bar, vorzugsweise zwischen 8 und 12 bar, arbeitet.

3. Verfahren nach Anspruch 1 oder 2, in dem die Reaktionstemperatur zwischen 10 und 90 °C liegt und durch Einspritzen von flüssigem SO₂ in die Reaktionszone konstant gehalten wird.

4. Verfahren nach einem der Ansprüche 1 bis 3, in dem das Alkan Methan ist, wobei das dem Reaktor zugeführte Gasgemisch 1 bis 12 Mol Schwefeldioxid, 0,1 bis 1 Mol Chlor und 0,1 bis 0,6 Mol Chlorwasserstoff pro Mol Methan enthält.

5. Verfahren nach Anspruch 4, in dem das Gasgemisch 5 bis 7 Mol Schwefeldioxid, 0,7 bis 0,9 Mol Chlor und 0,4 bis 0,5 Mol Wasserstoffchlorid pro Mol Methan enthält.

6. Verfahren nach einem der Ansprüche 1 bis 3, in dem das Alkan mindestens 2 Kohlenstoffatome enthält, wobei das dem Reaktor zugeführte Gasgemisch 7 bis 14 Mol (vorzugsweise 10 bis 13 Mol) Schwefeldioxid und 0,1 bis 1 Mol (vorzugsweise 0,7 bis 0,9 Mol) Chlor pro Mol Alkan enthält.

## Claims

1. Process for the manufacture of alkanesulphonyl chlorides by photochemical reaction of an alkane with chlorine and sulphur dioxide, optionally in the presence of hydrogen chloride, **characterized in that** the light source used is a gallium-doped medium-pressure mercury lamp.

2. Process according to Claim 1, in which the operation is carried out at a pressure of from 1 to 15 bar relative, preferably between 8 and 12 bar relative.

3. Process according to Claim 1 or 2, in which the reaction temperature is between 10 and 90°C and is kept constant by injecting liquid SO₂ into the reaction zone.

4. Process according to one of Claims 1 to 3, in which the alkane is methane, the gas mixture fed to the reactor comprising 1 to 12 mol of sulphur dioxide, 0.1 to 1 mol of chlorine and 0.1 to 0.6 mol of hydrogen chloride per mole of methane.

5. Process according to Claim 4, in which the gas mixture contains 5 to 7 mol of sulphur dioxide, 0.7 to 0.9 mol of chlorine and 0.4 to 0.5 mol of hydrogen chloride per mole of methane.

6. Process according to one of Claims 1 to 3, in which the alkane contains at least two carbon atoms, the gas mixture fed to the reactor comprising 7 to 14 mol (preferably 10 to 13 mol) of sulphur dioxide and 0.1 to 1 mol of chlorine (preferably 0.7 to 0.9 mol) per mole of alkane.
